(19)

Europäisches
Patentamt

European
Patent Office

Office européen
des brevets

(11) EP 3 741 354 A1

(12) EUROPEAN PATENT APPLICATION

(43) Date of publication:
25.11.2020 Bulletin 2020/48

(51) Int Cl.:
A61K 8/73 (2006.01)      A61Q 17/04 (2006.01)

(21) Application number: 20169790.1

(22) Date of filing: 16.04.2020

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 23.05.2019 KR 20190060473

(71) Applicant: Nature Costech Co., Ltd.
Chungcheongbuk-do 28578 (KR)

(72) Inventors:
• JEON, So Young
  28650 Chungcheongbuk-do (KR)
• SONG, Woo Yong
  28374 Chungcheongbuk-do (KR)
• SHIN, Su Bok
  30100 Sejong-si (KR)

(74) Representative: Byrne, Declan
Andre Roland S.A.
Intellectual Property Services
P.O. Box 5107
1002 Lausanne (CH)

(54) **SUNSCREEN AGENT COMPRISING CELLULOSE NANOFIBERS**

(57) The present invention relates to a sunscreen agent comprising cellulose nanofibers, and discloses a use of cellulose nanofibers having a UV blocking effect as a sunscreen agent. The cellulose nanofibers according to the present invention is an organic sunscreen agent derived from natural plants, and has a UV scattering mechanism, which is a sunscreen mechanism of an inorganic sunscreen agent, and thus has characteristics that are harmless to the human body or skin. Therefore, it may be applied as a sunscreen cosmetic, and is preferably used as a biomaterial for an additive in a synthetic resin composition such as a pharmaceutical composition for skin, a film and the like.

**Description**

RELATED APPLICATIONS

This application claims priority to Korean Patent Application No. 10-2019-0060473, filed on May 23, 2019 in the Korean Intellectual Property Office, the entire disclosure of which is incorporated herein by reference.

Field of the Invention

The present invention relates to a sunscreen agent comprising cellulose nanofibers, and more specifically, to a sunscreen agent comprising cellulose nanofibers having an ultraviolet (UV) blocking effect as an effective ingredient.

BACKGROUND OF THE INVENTION

Description of the Related Art

Sunlight is a ray having multiple wavelengths, and is an inevitable ray for people or objects exposed to an outside.
A skin of a human is sensitive to sunlight having wavelengths between about 290 nm and 400 nm. In particular, an ultraviolet B (UV-B) region having a wavelength between about 290 nm and 320 nm is known to cause skin damages accompanying redness, erythema, edema or the like. Prolonged or continuous exposure to the ultraviolet rays ('UV rays') having the wavelength in the above-described range leads to severe skin diseases. In addition, an ultraviolet A (UV-A) region having a wavelength between about 320 nm and 400 nm is known to promote skin aging.
Further, when various materials constituting objects other than the skin of humans are exposed to the outside for a long period of time, due to changes in physical properties caused by the UV rays, a shortened lifespan or deformation occurs in the objects, and thereby the material often does not maintain original functions thereof.
Accordingly, recent sunscreen agents have been developed for obtaining a UV blocking effect through scattering, reflection, or absorption of the UV rays upon exposure to such UV rays.
Thereby, the sunscreen agents developed until recently have been studied for protection against UV-A and UV-B from a broad or narrow range of UV rays, retentive capacity relating to waterproofing and diaphoretic properties, ease of application, invisibility, non-contamination, ease of use such as slipping, and skin toxicity and skin safety caused by sunscreen ingredients and additives.
Effectiveness of the protection from sunlight is measured using a sun protection factor (SPF). Thereby, the sunscreen agents having SPFs developed until recently have increased public awareness of risks related to exposure to sunlight. Accordingly, a sunscreen agent having a high SPF is required.
The current sunscreen agents require quantitative UV protection. Typically, zinc oxide and titanium dioxide are inorganic sunscreen materials that provide UV protection effects such as UV scattering and refraction, and oxymethylcinnamate, octocrylene, and the like are organic sunscreen materials that provide chemical UV protection effects.
In general, the sunscreen agents are divided into inorganic sunscreen agents and organic sunscreen agents depending on sunscreen mechanisms of inorganic materials and organic materials contained therein. Among them, the inorganic sunscreen agent is applied with UV scattering as a main mechanism, and the organic sunscreen agent is applied with UV absorption as the main mechanism.
As conventional inorganic sunscreen agents, Korean Patent Publication Laid-Open No. 10-2009-0070454 proposes a sunscreen cosmetic composition which does not contain an organic sunscreen agent, and Korean Patent Publication Laid-Open No. 10-2017-0062126 proposes an emulsion type cosmetic composition comprising an inorganic sunscreen agent and a method for manufacturing the same.
However, the inorganic sunscreen agent is expensive, and when applied to the skin, white cloudiness occurs and feeling of use is poor. In addition, the inorganic sunscreen agent causes skin irritation due to reactive oxygen species (ROS), and when used alone, it is difficult to obtain a high sunscreen index, and thereby requiring a large amount in use.
Further, examples of the organic sunscreen agent include polymer composite particles comprising a large amount of organic sunscreen agents and a method for manufacturing the same proposed in Korean Patent Registration No. 10-1833612 and the like.
However, in the case of the organic sunscreen agent, a product made by photoreaction causes skin inflammation, generates cancer cells, and leads to side effects such as DNA mutation and infertility by absorbing UV rays, thereby causing safety problems in a human body.
Therefore, the existing inorganic sunscreen agents and organic sunscreen agents involve irritation to the skin or the human body and need to solve harmful problems due to characteristics thereof, and also have a disadvantage of a deterioration in functionality due to a narrow sunscreen region. Therefore, there is an urgent need for the development of alternatives that can overcome the above-described problems.

Prior Art Document

Patent Documents

1. Korean Patent Publication Laid-Open No. 10-2009-0070454

2. Korean Patent Publication Laid-Open No. 10-2017-0062126

3. Korean Patent Registration No. 10-1833612

SUMMARY OF THE INVENTION

In consideration of the above-mentioned circumstances, it is a challenge of the present invention to develop a new sunscreen agent capable of solving all problems entailed in existing organic sunscreen materials and inorganic sunscreen materials.

Accordingly, it is an object of the present invention to provide a new use of cellulose nanofibers having a UV scattering mechanism, which is a sunscreen mechanism of an inorganic sunscreen agent, as a natural organic material-derived sunscreen agent.

In addition, another object of the present invention is to provide a sunscreen agent comprising cellulose nanofibers, which is safer to a human body compared to conventional products due to a use of naturally-derived ingredients and a scattering effect rather than light absorption, while having superior physical properties as a sunscreen agent compared to the conventional products.

To achieve the above-described objects, according to an aspect of the present invention, there is provided a cellulose nanofiber having UV blocking abilities, and an average diameter of 5 to 50 nm and an average length of 100 to 900 nm.

According to a preferred embodiment of the present invention, the cellulose nanofibers have an average diameter of 10 to 40 nm, preferably 10 to 30 nm, more preferably 15 to 30 nm, and most preferably 15 to 25 nm.

According to a preferred embodiment of the present invention, the cellulose nanofibers may include carboxymethyl cellulose nanofibers, TEMPO(2,2,6,6-tetramethyl-1-piperidinyloxy, 2,2,6,6-tetramethylpiperidine 1-oxyl free radical, (2,2,6,6-tetramethylpiperidin-1-yl)oxyl or (2,2,6,6-tetramethylpiperidin-1-yl)oxidanyl)-oxidized cellulose nanofibers, or a mixture thereof.

Herein, the carboxymethyl cellulose nanofiber preferably has a degree of substitution (DS) by a carboxymethyl group of 0.05 to 0.3.

Further, the TEMPO-oxidized cellulose nanofiber preferably has a degree of oxidation (DO) by oxidant such as sodium hypochlorite (NaClO), sodium hypobromite (NaBrO) or a mixture thereof of 0.01 to 0.3. Furthermore, the TEMPO-oxidized cellulose nanofiber may have an amount of used oxidant per 1 g of cellulose of 0.1 to 3 mM/g.

In addition, according to a preferred embodiment of the present invention, the cellulose nanofibers have unabsorbent UV blocking abilities on an ultraviolet A region having a wavelength of 315 to 400 nm and an ultraviolet B region having a wavelength of 280 to 315 nm.

According to an aspect of the present invention, there is provided a sunscreen agent comprising cellulose nanofibers which have UV blocking abilities, and an average diameter of 5 to 50 nm and an average length of 100 to 900 nm, as an effective ingredient.

In addition, according to an aspect of the present invention, there is provided a composition for a skin care sunscreen agent comprising cellulose nanofibers.

Further, according to an aspect of the present invention, there is provided a skin care sunscreen cream comprising the sunscreen agent.

Further, according to an aspect of the present invention, there is provided a cosmetic composition comprising the sunscreen agent.

Further, according to an aspect of the present invention, there is provided a pharmaceutical composition for skin comprising the sunscreen agent.

Furthermore, according to an aspect of the present invention, there is provided a synthetic resin composition comprising the sunscreen agent.

According to the present invention, the cellulose nanofibers as described above have their own UV scattering effects and have a harmless characteristic to the human body or skin, thus may be utilized as biomaterials such as sunscreen cosmetics and pharmaceutical products. In addition, the cellulose nanofibers may be utilized as a material for synthetic resin additives used in a production of films or sheets having UV blocking abilities.

In particular, the cellulose nanofiber of the present invention is a naturally-derived biomaterial, and may be used as a material having a function of adjusting a viscosity in various human-friendly compositions, as well as a new functionality and a use to block UV rays due to UV scattering without absorbing UV rays, thereby having very useful effects as an eco-friendly and bio-friendly biomaterial.

The effects of the present invention as described above are not limited thereto, and other effects of the present invention will be clearly understood by persons having a common knowledge in the technical field to which the present invention pertains through examples and claims, which will be described below.

DETAILED DESCRIPTION OF THE INVENTION

Hereinafter, the present invention will be described in more detail.

The present invention relates to a sunscreen agent comprising cellulose nanofibers having a UV blocking effect, and discloses a use of cellulose nanofibers as a sunscreen material.

According to the present invention, the cellulose nanofibers are provided as an organic material-derived sunscreen agent, as well as a natural-friendly and eco-friendly biomaterial derived from natural products having a UV scattering mechanism, which is a UV blocking mechanism of an inorganic sunscreen agent.

According to a preferred embodiment of the present invention, specifically, the cellulose nanofibers of the present invention are characterized by having an average diameter of 5 to 50 nm and an average length of 100 to 900 nm. Herein, when the average diameter of the cellulose nanofibers is too large or small, the UV blocking effect due to the UV scattering effect cannot be expected. In particular, if the average diameter or the average length thereof is too large, a size of the cellulose nanofiber particle becomes too large, and when applying it to a skin or the like, there may be feeling of poor texture, etc., thus it is not preferable.

According to a preferred embodiment of the present invention, the cellulose nanofibers used herein may be carboxymethyl cellulose nanofibers substituted with a carboxymethyl group. Alternatively, TEMPO-oxidized cellulose nanofibers oxidized by TEMPO also have a UV blocking effect. In addition, cellulose nanofibers made of a mixture thereof may also be used as a material for blocking UV rays.

According to a preferred embodiment of the present invention, the carboxymethyl cellulose nanofibers preferably used herein may have an average degree of substitution by a carboxymethyl group of 0.05 to 0.3. If the degree of substitution is less than 0.05, it is not preferable to use as a sunscreen agent used for skin application due to poor physical properties. If the degree of substitution thereof exceeds 0.3, the UV blocking effect is rapidly decreased.

In addition, according to a preferred embodiment of the present invention, the TEMPO-oxidized cellulose nanofibers preferably have an average degree of oxidation by a TEMPO free radical in a range of 0.01 to 0.3. Herein, if the degree of oxidation thereof is too small or too large beyond the above range, it is difficult to expect a blocking effect due to UV scattering. To this end, according to the present invention, the TEMPO-oxidized cellulose nanofibers preferably have an oxidation amount per 1 g of cellulose by a TEMPO free radical and an oxidant of 0.1 to 3 mM/g, and more preferably 0.04 to 1.5 mM/g.

The cellulose nanofibers satisfying the above-described conditions according to the present invention exhibit UV blocking effects on an ultraviolet A region having a wavelength of 315 to 400 nm and an ultraviolet B region having a wavelength of 280 to 315 nm.

Particularly, it has been confirmed that the cellulose nanofibers having the above-described specific conditions according to the present invention preferably exhibit effects of blocking UV rays due to a scattering mechanism of UV rays without absorbing UV rays. As described above, the cellulose nanofibers according to the present invention have a characteristic of not absorbing UV rays despite being organic materials. This is a very surprising effect newly discovered by the present inventors, and exhibits a completely different characteristic from the existing organic sunscreen material.

According to the present invention, the carboxymethyl cellulose nanofibers may be prepared by, for example, adding a chloroacetic acid to an anionized pulp, then performing a reaction, and cutting it into a length of 100 to 900 nm. In this manufacturing process, a cutting effect may be obtained by simultaneously using a grinder and a homogenizer. By carrying out these grinding and homogenization processes several times, preferably 2 to 6 times, carboxymethyl cellulose nanofibers having desirable properties may be prepared.

In addition, the TEMPO-oxidized cellulose nanofibers may be prepared by a conventional method of oxidizing cellulose with sodium hypochlorite, sodium hypobromite or a mixture thereof by using a TEMPO, followed by grinding and homogenization.

The above-described cellulose nanofibers according to the present invention exhibit a UV blocking effect due to excellent UV scattering with only a single component without the help of other organic or inorganic sunscreen agents. Surprisingly, it has been confirmed that the cellulose nanofibers according to the present invention have excellent UV blocking effects while absorbing very little UV rays, unlike other existing organic sunscreen materials.

The cellulose nanofibers according to the present invention are obtained from natural plant-derived ingredients, and have harmless characteristics to the human body or skin, and thereby may be safely applied to the human body in natural-friendly, eco-friendly, and human-friendly manners.

Therefore, the cellulose nanofiber according to the present invention may be widely utilized as a biomaterial applicable to the human body, such as for cosmetics and pharmaceutical products for blocking UV rays, and may be utilized as an additive material of a synthetic resin composition for manufacturing films or sheets.

In particular, the cellulose nanofiber of the present invention is a natural plant-derived biomaterial, and has a function of adjusting a viscosity in various human-friendly compositions, thereby exhibiting an amazing effect of blocking UV rays due to scattering without absorbing the UV rays. For this reason, these cellulose nanofibers are not harmful to the human body, and therefore may be used as additives for sunscreens having a new functionality and a use, and may be very usefully used as eco-friendly and bio-friendly biomaterials.

Accordingly, the present invention provides a sunscreen agent comprising cellulose nanofibers having UV blocking abilities, and an average diameter of 5 to 50 nm and an average length of 100 to 900 nm, as an effective ingredient.

According to a preferred embodiment of the present invention, when the cellulose nanofibers are carboxymethyl cellulose nanofibers, it is preferable to have an average length of 500 to 900 nm, and more preferably 650 to 850 nm in consideration of manufacturing conditions and the like.

In addition, according to a preferred embodiment of the present invention, when the cellulose nanofibers are TEMPO-oxidized cellulose nanofibers, it is also preferable to have an average length of 100 to 500 nm, and more preferably 100 to 400 nm in consideration of the manufacturing conditions and the like.

Further, the cellulose nanofibers of the present invention may be prepared as a composition for a skin care sunscreen agent in various formulations, along with components for various uses comprising the sunscreen agent.

Typically, the present invention may provide a skin care sunscreen agent as a functional cosmetic comprising the sunscreen agent. The typical composition for a skin care sunscreen agent may be prepared by a conventional method as a composition for a sunscreen agent in a form of a slurry, cream, and the like, which is a commercially available product in general as a functional cosmetic. Typically, the composition may be prepared as a skin care sunscreen cream, which is a functional cosmetic.

According to the present invention, as an example, in the case of manufacturing the skin care sunscreen cream, which is a functional cosmetic, the skin care sunscreen agent may contain the cellulose nanofibers of the present invention in an amount of 0.1 to 10% by weight ('wt.%') based on a total weight of the composition.

In addition, the sunscreen agent of the present invention is cosmetics containing the sunscreen agent, and may be prepared and used as a cosmetic composition that can be mixed with the functional cosmetic or general cosmetic having functionalities such as whitening, moisturizing, wrinkle improvement or improvement of atopic dermatitis, and can be applied to the skin. In the case of such a cosmetic composition, the sunscreen agent is also preferably prepared by mixing with cream, lotion, skin lotion, essence, etc., then blending with other cosmetics in a conventional manner. Further, the sunscreen agent may be applied to cosmetics also having a UV blocking effect. When applying the sunscreen agent to such cosmetics by mixing as an additive, the cellulose nanofibers of the present invention may be used in an amount of 0.1 to 5 wt.%.

In addition to the cosmetics, the sunscreen agent of the present invention may be applied to a pharmaceutical product also having a UV blocking effect by mixing with various pharmaceutical compositions for skin used for treatment or prevention of skin diseases. Therefore, the sunscreen agent of the present invention may be preferably applied to such a pharmaceutical composition for skin as an additive for sunscreen.

When applying the sunscreen agent comprising cellulose nanofibers of the present invention to the pharmaceutical product as an additive for sunscreen, for example, the sunscreen agent may be applied to a skin ointment, a cream agent, etc., and may be used in an amount of 0.01 to 5 wt.% based on the total weight of the composition. In particular, it is preferable that the sunscreen agent comprising cellulose nanofibers of the present invention is used for treating skin weakness, scalds, injury, surgical damage requiring skin recovery, skin diseases that involve skin trouble, etc., and skin injury, and is very usefully used as an additive when protecting the skin from UV rays during healing a wound or normally regenerating the skin. In this case, when using the sunscreen agent in a manner of mixing with the pharmaceutical composition and applying to the skin one to three times a day or frequently by using a conventional method of using the pharmaceutical product for skin, it preferably functions as an additive for sunscreen to help original therapeutic effects of the pharmaceutical product. Such a cellulose nanofiber of the present invention is a non-toxic component and may be widely used without side effects on the skin.

Meanwhile, the sunscreen agent according to the present invention may be used by mixing it into a synthetic resin composition that requires the UV blocking effect as an organic sunscreen material which is a natural plant material.

As an example thereof, in order to prevent UV exposure of the skin from sunlight, the sunscreen agent may be mixed with a synthetic resin composition for manufacturing films such as functional or non-functional transparent, translucent, and opaque films so as to provide the UV blocking effect. In particular, since the cellulose nanofiber of the present invention has transparency, it is preferably applied to typical transparent or translucent UV blocking films.

In addition, similarly, the cellulose nanofibers of the present invention may be applied by mixing with a sheet type synthetic resin material as an additive for sunscreen, similar to the film.

The sunscreen agent according to the present invention is applied to various synthetic resin molded products as an additive other than the films or sheets, when requiring to prevent UV rays from sun rays indoors or outdoors, or in order to prevent changes in physical properties, changes in functions, and a deterioration in durability of materials due to UV rays.

In particular, the sunscreen agent comprising the cellulose nanofibers of the present invention is a natural material obtained from natural plants, and thus has biodegradability. Therefore, it is very preferable to apply to a biodegradable synthetic resin composition or a synthetic resin composition applied to the human body.

In addition, the sunscreen agent of the present invention may be utilized in various ways as a synthetic resin composition for manufacturing various molded products, such as for a film or sheet containing the same.

When using the sunscreen agent as a material for adding such a synthetic resin, the cellulose nanofibers of the present invention are mixed in an amount of 0.1 to 10 wt.% to a synthetic resin such as polypropylene, polyethylene, PVC, nylon, cellophane, and EVA, or a synthetic resin composition such as a conventional biodegradable resin. At this time, the cellulose nanofibers may be made of a composition in consideration of a form according to an application and a purpose, and may be prepared in a conventional plastic molding method.

As described above, the cellulose nanofiber according to the present invention is an organic sunscreen agent derived from natural plant resources, and has a UV scattering mechanism, which is a sunscreen mechanism of the inorganic sunscreen agent, and thus has an unabsorbent UV blocking effect. Therefore, the cellulose nanofibers have characteristics that they are harmless to the human body or skin.

Accordingly, the cellulose nanofibers of the present invention may be applied to sunscreen cosmetics, and preferably, may be widely used as a biomaterial for addition to pharmaceutical compositions for skin and synthetic resin compositions such as films and sheets.

Hereinafter, examples of the present invention will be described in more detail. However, the following examples are only intended to specifically illustrate the contents of the present invention, and the present invention is not limited thereto.

Example 1: Process for carboxymethylation of cellulose

NaOH was dissolved in 95% of EtOH in advance, then a mixture of pulp, NaOH and EtOH was added to a reaction vessel, mixed well, and immersed for 1 hr at room temperature.

Monochloroacetate (MCA) and 95% of EtOH were added and dissolved, then a mixture of NaOH and EtOH was put into the reaction vessel and mixed well.

A reaction was performed for 2 hrs at 75 °C in an oven, followed by washing after the reaction was completed until pH reached 7.

<Used reagent>

- NaOH: Sodium hydroxide, 98.0% (SAMCHUN CHEMICALS)
- MCA: Sodium chloroacetate, 98.0% (SAMCHUN CHEMICALS)

<Equation for adjusting degree of substitution in carboxymethylation pre-treatment process>

A weight of the used component is determined according to a degree of substitution by using Equation 1 below to perform a pre-treatment process.

[Equation 1]

Number of moles of 100 g of pulp: 100 / 162 (Molecular weight of cellulose) = 0.617284

Amount of MCA = Number of moles of cellulose (100 / 162) × Degree of substitution (adjustment) × 117 (Molecular weight of sodium chloroacetate)

Amount of NaOH = Number of moles of cellulose (100 / 162) × Degree of substitution (adjustment) × 40 (Molecular weight of NaOH)

<Amount (g) of used component for each degree of substitution in carboxymethylation pre-treatment process>

Amounts of the used each component in the cellulose pre-treatment process as described above for each degree of substitution were summarized, and the results thereof are listed in Table 1 below.

Table 1

| DS | 0.05 | 0.1 | 0.15 | 0.2 | 0.25 | 0.3 | 0.4 | 0.5 | 0.6 |
|------|------|------|-------|-------|-------|-------|-------|-------|-------|
| Pulp | 100 | 100 | 100 | 100 | 100 | 100 | 100 | 100 | 100 |
| MCA | 3.61 | 7.22 | 10.83 | 14.44 | 18.05 | 21.66 | 28.88 | 36.11 | 43.33 |
| NaOH | 1.23 | 2.46 | 3.69 | 4.93 | 6.15 | 7.40 | 9.87 | 12.34 | 14.81 |

Example 2: TEMPO-oxidation treatment process for cellulose

After dispersing the pulp and distilled water, catalysts (TEMPO, NaBr) were dissolved, and after the catalyst was completely dissolved, NaClO was added, and NaOH was added whenever the pH dropped to 10.5 or less to maintain the pH.
Subsequently, when the pH remained unchanged for about 30 minutes, washing was performed.

<Used reagent>

- NaBr: Sodium bromide, 99,0% (SAMCHUN CHEMICALS)
- TEMPO: TEMPO 98% (Sigma Aldrich)
- NaClO: 5.54% NaClO (Songi Lax)

<Equation for adjusting degree of oxidation in TEMPO-oxidation process>

100 g of pulp was weighed, and a catalyst of an oxidation-reduction reaction was weighed (TEMPO 0.5 g, NaBr 2.5 g).
Then, a NaClO sample was weighed to a desired extent to be oxidized by using Equation 2 below.

[Equation 2]

(Amount of NaClO sample) × 0.054 (Concentration of NaClO

5.4%) ÷ 73 (Molecular weight of NaClO) ÷ 100 (Weight of pulp)

= 1.25 mM (Number of moles for desired degree of oxidation)

<Amount (g) of used components according to the number of moles of TEMPO in TEMPO-oxidation process>

Amounts of the used each component according to the number of moles of oxidation amount (mM) in the TEMPO-oxidation process as described above were summarized, and the results thereof are listed in Table 2 below.

Table 2

| Oxidation amount (mM) | 0.5 | 0.7 | 1 | 1.25 | 1.5 | 2 | 3 | 5 |
|---|---|---|---|---|---|---|---|---|
| Pulp | 100 | 100 | 100 | 100 | 100 | 100 | 100 | 100 |
| NaClO | 67.59 | 94.62 | 135.18 | 168.98 | 202.27 | 135.18 | 405.54 | 675.9 |

Experimental Example 1: UV transmittance experiment of carboxymethyl cellulose nanofibers

Carboxymethyl cellulose nanofibers having various degrees of substitution were prepared according to Example 1, and UV transmittance (%) of each of carboxymethyl cellulose nanofibers was measured under the same conditions based on a sample concentration of 1.5% aqueous solution for each degree of substitution (DS), and the results thereof are listed in Table 3 below.
In this experiment, the UV transmittance test was performed by an ISO TS 17466:2015 method for each sample.

Table 3

| DS | 0.05 | 0.1 | 0.15 | 0.2 | 0.25 | 0.3 | 0.4 | 0.5 | 0.6 |
|---|---|---|---|---|---|---|---|---|---|
| UVA 350 (nm) | 50.3 | 60.8 | 41.8 | 29.3 | 59.6 | 71.4 | 84.8 | 89 | 80.2 |
| UVB 300 (nm) | 42.1 | 55.5 | 37.3 | 25.5 | 57.2 | 67.5 | 83.5 | 88.2 | 77.9 |

Experimental Example 2: Absorbance experiment of carboxymethyl cellulose nanofibers

For an absorbance experiment of the carboxymethyl cellulose nanofibers prepared according to Example 1, absorbance (abs) of each of the carboxymethyl cellulose nanofibers having various degrees of substitution (DS) under the same conditions based on a sample concentration of 1.5% aqueous solution for each degree of substitution was measured, and the results thereof are listed in Table 4 below.
In this experiment, the absorbance experiment was performed by an ISO TS 17466:2015 method for each sample.

Table 4

| DS | 0.05 | 0.1 | 0.15 | 0.2 | 0.25 | 0.3 | 0.4 | 0.5 | 0.6 |
|---|---|---|---|---|---|---|---|---|---|
| UVA 350 (nm) | 0.432 | 0.405 | 0.343 | 0.305 | 0.271 | 0.22 | 0.1 | 0.085 | 0.185 |
| UVB 300 (nm) | 0.477 | 0.479 | 0.412 | 0.351 | 0.315 | 0.297 | 0.106 | 0.09 | 0.237 |

Experimental Example 3: UV transmission experiment of TEMPO-oxidized cellulose nanofibers

For a UV transmittance experiment of the TEMPO-oxidized cellulose nanofibers prepared in Example 2, UV transmittance (%) of each of the TEMPO-oxidized cellulose nanofibers was measured under the same conditions based on a sample concentration of 1.5% aqueous solution according to an amount of used oxidant per 1 g of cellulose (mM/g) and a degree of oxidation (DO), and the results thereof are listed in Table 5 below.

In this experiment, the UV transmittance experiment was performed by an ISO TS 17466:2015 method for each sample.

Table 5

| TEMPO (mM) | 0.5 | 0.7 | 1 | 1.25 | 1.5 | 2 | 3 | 5 |
|---|---|---|---|---|---|---|---|---|
| UVA 350 (nm) | 50 | 53.4 | 66.3 | 69.6 | 79.7 | 74.6 | 78.1 | 87.1 |
| UVB 300 (nm) | 41.4 | 49.7 | 64.2 | 67.1 | 77.8 | 72.7 | 76.5 | 86.7 |

Experimental Example 4: Absorbance experiment of TEMPO-oxidized cellulose nanofibers

For an absorbance experiment of the TEMPO-oxidized cellulose nanofibers prepared according to Example 2, absorbance (abs) of each of the TEMPO-oxidized cellulose nanofibers was measured under the same conditions based on a sample concentration of 1.5% aqueous solution according to an amount of used oxidant per 1 g of cellulose (mM/g) and a degree of oxidation (DO), and the results thereof are listed in Table 6 below.
In this experiment, the UV transmittance test was performed by an ISO TS 17466:2015 method for each sample.

Table 6

| TEMPO (mM) | 0.5 | 0.7 | 1 | 1.25 | 1.5 | 2 | 3 | 5 |
|---|---|---|---|---|---|---|---|---|
| UVA 350 (nm) | 0.306 | 0.301 | 0.176 | 0.165 | 0.152 | 0.123 | 0.101 | 0.099 |
| UVB 300 (nm) | 0.342 | 0.326 | 0.202 | 0.174 | 0.163 | 0.156 | 0.106 | 0.095 |

Experimental Example 5: Experiment of relationship between width and length according to degree of substitution

A distribution for widths (diameters) and lengths of each of the carboxymethyl cellulose nanofibers prepared by Example 1 according to degrees of substitution from 0.1 to 0.6 was measured, and the results thereof are listed in Table 7 below.
Herein, a distribution range of a size (X%) means that the width (diameter) or the length of the cellulose unit falls within the corresponding numerical range.

Table 7

| | | Width (nm) | | | Length (nm) | | |
|---|---|---|---|---|---|---|---|
| Distribution range of size (%) | | $X \leq 10$ | $10 < X \leq 50$ | $50 < X \leq 90$ | $X \leq 10$ | $10 < X \leq 50$ | $50 < X \leq 90$ |
| Degree of substitution (DS) | 0.1 | 37.4 | 41.8 | 46.4 | 633.7 | 740.2 | 846.4 |
| | 0.2 | 12.3 | 12.8 | 13.7 | 695.5 | 751.0 | 761.2 |
| | 0.3 | 8.0 | 8.7 | 9.1 | 681.8 | 727.8 | 755.1 |
| | 0.4 | 6.9 | 7.2 | 7.6 | 673.3 | 716.5 | 762.2 |
| | 0.5 | 8.6 | 8.9 | 9.5 | 652.7 | 820.0 | 916.7 |
| | 0.6 | 3.9 | 4.0 | 4.3 | 564.5 | 604.5 | 612.4 |

Experimental Example 6: Experiment of relationship between width and length according to amount of used oxidant and degree of oxidation

A distribution for widths (diameters) and lengths of each of the TEMPO-oxidized cellulose nanofibers prepared by Example 2 according to amounts of used oxidant (NaClO) from 0.5 to 5 mM/(cellulose g) and degrees of oxidation (DO) from 0.04 to 0.34 was measured, and the results thereof are listed in Table 8 below.
Herein, a distribution range (X%) of a size means that the width (diameter) or the length of the cellulose unit falls within the corresponding numerical range.

Table 8

| Distribution range of size (%) | | Width (nm) | | | Length (nm) | | | Degree of oxidation (DO) |
|---|---|---|---|---|---|---|---|---|
| | | X≤10 | 10<X≤50 | 50<X≤90 | X≤10 | 10<X≤50 | 50<X≤90 | |
| Amount of used NaClO (mM/g) | 0.5 | 20.2 | 28.1 | 38.0 | 194.4 | 212.1 | 379.7 | 0.04 |
| | 0.7 | 17.0 | 20.9 | 26.3 | 148.1 | 202.6 | 336.8 | 0.05 |
| | 1.25 | 10.5 | 14.3 | 23.0 | 113.3 | 198.6 | 300.0 | 0.09 |
| | 2 | 9.5 | 12.8 | 22.0 | 106.1 | 189.5 | 263.2 | 0.15 |
| | 3 | 8.5 | 12.3 | 17.2 | 80.7 | 168.4 | 238.8 | 0.23 |
| | 5 | 4.8 | 6.1 | 8.8 | 49.3 | 83.8 | 123.1 | 0.34 |

Terms used in the above Examples and Experimental Examples are defined as follows:

(1) The degree of substitution (DS) represents degrees (minimum 0, maximum 3) in which the cellulose nanofibers are substituted with a carboxymethyl group as an average value;

(2) The degree of oxidation (DO) represents degrees (minimum 0, maximum 1) in which the cellulose nanofibers are subjected to oxidization treatment as an average value;

(3) The blocking effect is a numerical value representing degrees of UV blocking in UV regions in the UV transmittance experiment according to the experimental examples;

(4) An effect on absorbance is a numerical value representing degrees in which the sample absorbs light in a UV wavelength range in the ultraviolet absorption experiment according to the experimental examples; and

(5) UVA and UVB represent transmission or absorption results at 350 nm and 300 nm, respectively.

Reviewing the experimental results of Experimental Examples 1 to 4, it has been confirmed that the cellulose nanofibers of the Examples 1 and 2 (having an average width of 5 to 50 nm, and an average length of 100 to 900 nm) according to the present invention exhibited excellent UV blocking effects in the UV transmittance experiment (Experimental Examples 1 and 3), and shown that there was almost no absorption in the ultraviolet absorption experiment (Experimental Examples 2 and 4). As a result, it has been confirmed that the cellulose nanofibers are preferably usable as a sunscreen agent having excellent UV blocking effects due to only the UV scattering mechanism without UV absorption.

Through the experimental results of these examples and experimental examples, it can be confirmed that the cellulose nanofibers of the organic natural materials according to the present invention are very useful as a sunscreen material.

In particular, reviewing the results of these experiments, as UV blocking effects on the aqueous solution of the cellulose nanofibers prepared in Example 1, it has been confirmed that the cellulose nanofibers exhibited UVA transmittances of 50.3 to 71.7% at the degrees of substitution (DS) of 0.05 to 0.3 by the carboxymethyl group to have UVA blocking effects of about 30 to 50%, and exhibited UVB transmittance of 42.1 to 76.5% to have UVB blocking effects of about 25 to 60% (Experimental Example 1). Further, it has been confirmed that the cellulose nanofibers exhibited an absorbance of 0.5% or less in the entire region to have no UV absorption (Experimental Example 2). Therefore, it has been confirmed that the cellulose nanofibers had the most preferable UV blocking effect without absorption at degrees of substitution (DS) of 0.05 to 0.3.

On the other hand, if the degree of substitution thereof is less than 0.05, the width and length thereof were too large, thereby making it difficult to use as an additive for cosmetics or pharmaceutical products. If the degree of substitution thereof exceeds 0.3, it has been confirmed that the UV blocking effect was rapidly decreased (Experimental Example 1).

As a result, it has been confirmed that, when the cellulose nanofibers had widths (diameters) of 5 to 50 nm and lengths of 900 nm or less, the UV blocking effect could be expected (Experimental Example 5).

In addition, it has been confirmed that the cellulose nanofibers prepared in Example 2 exhibited UVA transmittances of 50 to 79.7% at degrees of oxidation (DOs) by TEMPO-oxidation of 0.034 to 0.23 (amounts of used oxidant 0.5 to 3 mM/g) as a UV blocking effect on an aqueous solution thereof to have UVA blocking effects of about 20 to 50%, and exhibited UVB transmittances of 41.1 to 77.8% to have UVB blocking effects of about 22 to 60% (Experimental Example 3). Further, it has been confirmed that the cellulose nanofibers exhibited an absorbance of 0.5% or less in the entire region to have no UV absorption (Experimental Example 4). Therefore, it has been confirmed that the cellulose nanofibers had the most preferable UV blocking effect at degrees of oxidation (DOs) in a range of 0.01 to 0.3.

However, if the degree of oxidation thereof is less than 0.01, the width and length thereof were too large to inhibit the formulation, thereby making it difficult to use as an additive for cosmetics or pharmaceutical products. If the degree of oxidation thereof exceeds 0.3, it has been confirmed that the UV blocking effect was rapidly decreased (Experimental Example 3).

As a result, it has been confirmed that, when the cellulose nanofibers had widths (diameters) of 5 to 50 nm and lengths of 100 nm or more, the UV blocking effect could be expected (Experimental Example 5).

Summarizing the results of these experiments, the cellulose nanofibers of the present invention exhibit excellent UV blocking effects when they have an average width (diameter) of 5 to 50 nm and an average length of 100 to 900 nm. Since such a UV blocking effect has almost no absorbance and only a UV blocking effect, it has been confirmed that it is a UV blocking effect due to the scattering mechanism of UV rays. Therefore, it has been confirmed that the cellulose nanofiber of the present invention is very useful as a sunscreen agent by itself.

As described above, in an environment in which it is very important to protect a skin from UV rays irradiated from sunlight due to the recent increased environmental pollution and destruction of an ozone layer caused by exhaust gas, since the sunscreen agent comprising cellulose nanofibers of the present invention is derived from natural plants, it is expected to be very useful for cosmetics, pharmaceutical products, films, sheets, biodegradable resins, and synthetic resin compositions for molding, and the like, as a natural-friendly and human-friendly natural biomaterial.

**Claims**

1. A cellulose nanofiber having UV blocking abilities, and an average diameter of 5 to 50 nm and an average length of 100 to 900 nm.

2. A sunscreen agent comprising cellulose nanofibers which have UV blocking abilities, and an average diameter of 5 to 50 nm and an average length of 100 to 900 nm, as an effective ingredient.

3. The sunscreen agent according to claim 2, wherein the cellulose nanofibers include carboxymethyl cellulose nanofibers substituted with a carboxymethyl group, TEMPO-oxidized cellulose nanofibers oxidized by TEMPO or a mixture thereof.

4. The sunscreen agent according to claim 3, wherein the carboxymethyl cellulose nanofibers have an average degree of substitution by a carboxymethyl group of 0.05 to 0.3.

5. The sunscreen agent according to claim 3, wherein the carboxymethyl cellulose nanofibers have an average length of 500 to 900 nm.

6. The sunscreen agent according to claim 3, wherein the TEMPO-oxidized cellulose nanofibers have an average degree of oxidation of 0.01 to 0.3, or an amount of used oxidant per 1 g of cellulose of 0.1 to 3 mM/g.

7. The sunscreen agent according to claim 3, wherein the TEMPO-oxidized cellulose nanofibers have an average length of 100 to 500 nm.

8. The sunscreen agent according to claims 2, wherein the cellulose nanofibers further have unabsorbent UV blocking abilities on an ultraviolet A region having a wavelength of 315 to 400 nm and an ultraviolet B region having a wavelength of 280 to 315 nm.

9. The sunscreen agent according to claims 2, for using a composition for a skin care sunscreen agent.

10. The sunscreen agent according to claims 9, wherein the composition for a skin care sunscreen agent is a skin care sunscreen cream, or a cosmetic composition or a pharmaceutical composition for skin as an additive.

11. The sunscreen agent according to claims 2, for using a synthetic resin composition as an additive.

12. The sunscreen agent according to claims 11, wherein the synthetic resin composition is a composition for a film or sheet.

Europäisches
Patentamt

European
Patent Office

Office européen
des brevets

# EUROPEAN SEARCH REPORT

Application Number

EP 20 16 9790

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/033833 A1 (BIOVISION TECHNOLOGY INC [CA]; SAVAGE FRANEY ANNE [CA] ET AL.) 14 March 2013 (2013-03-14) * paragraphs [0015] - [0020]; claims 1-39 * <br> * example 10 * | 1-12 | INV. A61K8/73 A61Q17/04 |
| X | US 2015/297469 A1 (HAYASHI HISATO [JP] ET AL) 22 October 2015 (2015-10-22) * paragraphs [0083] - [0125]; tables 1-2 * | 1,2,8-10 | |
| X | JP 2017 109986 A (DAI ICHI KOGYO SEIYAKU CO LTD) 22 June 2017 (2017-06-22) * paragraphs [0020], [0027], [0035], [0069], [0073], [0081]; claim all; table 1 * <br> * paragraphs [0092], [0093] * | 1-12 | |
| X,P | WO 2020/004729 A1 (NATURE COSTECH CO LTD [KR]) 2 January 2020 (2020-01-02) * paragraphs [0140], [0158], [0159], [0168]; claims 1-9 * | 1-3,5, 8-10 | |
| A | DOUGLAS R. HAYDEN ET AL: "Fully Biobased Highly Transparent Nanopaper with UV-Blocking Functionality", ACS APPLIED POLYMER MATERIALS, vol. 1, no. 4, 7 March 2019 (2019-03-07), pages 641-646, XP55713366, ISSN: 2637-6105, DOI: 10.1021/acsapm.9b00192 * the whole document * | 1-12 | TECHNICAL FIELDS SEARCHED (IPC) <br><br> A61K A61Q |

-/--

The present search report has been drawn up for all claims

| Place of search | Date of completion of the search | Examiner |
|---|---|---|
| Munich | 13 July 2020 | Nopper, Agathe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHEN ZHANG ET AL: "Cinnamate-Functionalized Cellulose Nanocrystals as UV-Shielding Nanofillers in Sunscreen and Transparent Polymer Films", ADVANCED SUSTAINABLE SYSTEMS, vol. 3, no. 4, 29 April 2019 (2019-04-29), page 1800156, XP055713368, US ISSN: 2366-7486, DOI: 10.1002/adsu.201800156 * the whole document * | 1-12 | |
| A | EP 3 130 435 A1 (TECHNICAL INST OF PHYSICS AND CHEMISTRY OF THE CHINESE ACAD OF SCIENCE) 15 February 2017 (2017-02-15) * the whole document * | 1-12 | |
| A | AKIRA ISOGAI ET AL: "TEMPO-oxidized cellulose nanofibers", NANOSCALE, vol. 3, no. 1, 1 January 2011 (2011-01-01), pages 71-85, XP055184316, ISSN: 2040-3364, DOI: 10.1039/C0NR00583E * the whole document * | 1-12 | |

TECHNICAL FIELDS
SEARCHED (IPC)

The present search report has been drawn up for all claims

| Place of search | Date of completion of the search | Examiner |
|---|---|---|
| Munich | 13 July 2020 | Nopper, Agathe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 16 9790

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2020

| Patent document cited in search report | | Publication date | Patent family member(s) | | Publication date |
|---|---|---|---|---|---|
| WO 2013033833 | A1 | 14-03-2013 | NONE | | |
| US 2015297469 | A1 | 22-10-2015 | CN | 104955440 A | 30-09-2015 |
| | | | EP | 2929874 A1 | 14-10-2015 |
| | | | JP | 2018109030 A | 12-07-2018 |
| | | | JP | WO2014088034 A1 | 05-01-2017 |
| | | | US | 2015297469 A1 | 22-10-2015 |
| | | | WO | 2014088034 A1 | 12-06-2014 |
| JP 2017109986 | A | 22-06-2017 | JP | 6276812 B2 | 07-02-2018 |
| | | | JP | 2017109986 A | 22-06-2017 |
| WO 2020004729 | A1 | 02-01-2020 | KR | 20200000579 A | 03-01-2020 |
| | | | WO | 2020004729 A1 | 02-01-2020 |
| EP 3130435 | A1 | 15-02-2017 | CN | 104974357 A | 14-10-2015 |
| | | | EP | 3130435 A1 | 15-02-2017 |
| | | | JP | 2017519890 A | 20-07-2017 |
| | | | US | 2017027842 A1 | 02-02-2017 |
| | | | WO | 2015154692 A1 | 15-10-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190060473
- KR 1020090070454
- KR 1020170062126
- KR 101833612